# EUROPEAN PATENT SPECIFICATION

(11) **EP 3 242 234 B1**
(45) Date of publication and mention of the grant of the patent: **04.11.2020**
(21) Application number: 17169331.0
(22) Date of filing: 03.05.2017
(51) Int. Cl.: G16H 40/63, G16H 20/40, G16H 40/20

(54) **SYSTEMS AND METHODS FOR SIMULATING PRIOR USE OF A SURGICAL INSTRUMENT BASED ON OBTAINED SURGICAL INSTRUMENT DATA**
SYSTEME UND VERFAHREN ZUM SIMULIEREN VOR DER BENUTZUNG EINES CHIRURGISCHEN INSTRUMENTS BASIEREND AUF ERHALTENEN DATEN DES CHIRURGISCHEN INSTRUMENTS
SYSTÈMES ET PROCÉDÉS DE SIMULATION D'UTILISATION PRÉALABLE D'UN INSTRUMENT CHIRURGICAL SUR LA BASE DES DONNÉES D'INSTRUMENT CHIRURGICAL OBTENUES

(30) Priority: 04.05.2016 US 201662331824 P; 19.04.2017 US 201715491046
(43) Date of publication of application: 08.11.2017
(60) Divisional application: 20194365.1
(73) Proprietor: Covidien LP, Mansfield, MA 02048 (US)
(72) Inventor: WINGARDNER, Thomas, North Haven, CT 06473 (US); COLLINGS, Peter T., Shelton, CT 06484 (US); DURANT, David, Wallingford, CT 06492 (US); IRKA, Philip, Madison, CT 06443 (US); VALENTINE, Kelly, New Britain, CT 06053 (US); INGMANSON, Michael, Stratford, CT 06615 (US)
(74) Representative: Maschio, Antonio

(56) References cited:
- WO-A1-2012/061718
- US-A1- 2011 121 049
- Peter I Corke: "A computer tool for simulation and analysis: the Robotics Toolbox for MATLAB", Proceedings of the 1995 National Conference of the Australian Robot Association, 1 July 1995 (1995-07-01), pages 319-330, XP055393158, Melbourne, Australia Retrieved from the Internet: URL:http://www.petercorke.com/RTB/ARA95.pd f [retrieved on 2017-07-24]

## Description

### BACKGROUND

### 1. Technical Field

The present disclosure relates to surgical instruments and, more particularly, to systems and methods for simulating prior use of a surgical instrument based on obtained surgical instrument data.

### 2. Background of Related Art

Many surgical instruments now incorporate hardware and software features that provide various benefits including facilitating operation and control of the instruments and/or components thereof, communication between the instruments and/or components thereof, sensor-based feedback between the instruments, components thereof, and/or surrounding environment, customization of the instruments and/or components thereof for a particular use, updating of the instruments and/or components thereof, etc. Such surgical instruments may further be configured to obtain and store data related to the instrument, components thereof, and/or surrounding environment such as, for example: identification data, type data, status data, and usage data.

While surgical instrument data such as that noted above is valuable for research and development, diagnostics, problem-solving, and other purposes, significant analysis, interpretation, correlation, and/or inferences are required to transform the raw data into useable information for these purposes. Thus, such data is only typically useable by those trained with the skills to interpret, correlate, and make inferences about the data.

US 2011/121049 discloses a surgical system and method that include a surgical instrument, configured to wirelessly transmit identifying data specific to the surgical instrument, and a console configured to receive the identifying data. The console is configured to register the surgical instrument based on the identifying data, establish a wireless two-way communication link between the surgical instrument and the console, receive at least one of operational data and commands from the surgical instrument, and provide operational feedback data to a user of the surgical instrument during an operation of the surgical instrument based on the at least one of operational data and commands.

### SUMMARY

To the extent consistent, any of the aspects and features of the present disclosure may be used in conjunction with any or all of the other aspects and features detailed herein.

Provided in accordance with aspects of the present disclosure is a surgical system including a surgical instrument, a processing component, and a display component. The surgical instrument includes a memory configured to store usage data from a prior use of the surgical instrument in one or more usage data files. The processing component is configured to receive the usage data file(s) from the surgical instrument and generate a simulation of the prior use of the surgical instrument based upon the usage data file(s). The display component is operably coupled to the processing component and configured to visually display the simulation of the prior use of the surgical instrument.

In an aspect of the present disclosure, the usage data stored in the usage data file(s) is time-stamped.

In another aspect of the present disclosure, the usage data includes keystroke data, sensor feedback data, and/or operational data.

In yet another aspect of the present disclosure, the surgical instrument includes a plurality of components configured to releasably engage one another. Each of the components is configured to generate component usage data for storage in the usage data file(s). The component usage data may be associated, in the usage data file(s), with identifying data of the component thereof.

In still another aspect of the present disclosure, the processing component and the display component are associated with a computer.

In aspects of the present disclosure, the simulation is visually displayed, via the display component, as a slideshow or as an animation.

In still yet another aspect of the present disclosure, the simulation further includes one or more icons indicating a proper or improper event in the prior use of the surgical instrument.

In another aspect of the present disclosure, the processing component is configured to group, manipulate, and/or correlate the usage data stored in the usage data file(s).

In yet another aspect of the present disclosure, the processing component is configured to associate the grouped, manipulated, and/or correlated usage data with an image or images used to generate the simulation.

A method provided in accordance with aspects of the present disclosure includes obtaining usage data from a prior use of a surgical instrument; storing the usage data in one or more usage data file(s), transmitting the usage data file(s), generating a simulation of the prior use of the surgical instrument based upon the usage data file(s), and displaying the simulation of the prior use of the surgical instrument.

In an aspect of the present disclosure, storing the usage data in the usage data file(s) includes time-stamping the usage data.

In another aspect of the present disclosure, obtaining the usage data includes obtaining keystroke data, sensor feedback data, and/or operational data.

In yet another aspect of the present disclosure, obtaining the usage data includes obtaining component usage data from a plurality of components of the surgical instrument. The method may further include storing the component usage data in association with identifying data of the component thereof.

In aspects of the present disclosure, displaying the simulation includes displaying a slideshow or displaying an animation.

In still another aspect of the present disclosure, displaying the simulation further includes displaying an icon indicating a proper or improper event in the prior use of the surgical instrument.

In still yet another aspect of the present disclosure, generating the simulation includes grouping, manipulating, and/or correlating the usage data stored in the usage data file(s), and associating the grouped, manipulated, and/or correlate usage data with an image or images.

### BRIEF DESCRIPTION OF THE DRAWINGS

The present disclosure and its various aspects and features are described hereinbelow with reference to the accompanying drawings, wherein:
FIG. 1 is a schematic illustration of a system provided in accordance with the present disclosure;
FIG. 2 is a schematic illustration of a surgical instrument configured for use with the system of FIG. 1;
FIGS. 3A-3D are images of a simulation representing the assembly of the surgical instrument of FIG 2, based on obtained surgical instrument data; and
FIGS. 4A-4E are images of a simulation representing the use of the surgical instrument of FIG 2, based on obtained surgical instrument data.

### DETAILED DESCRIPTION

The present disclosure relates to systems and methods for simulating prior use of a surgical instrument having a memory for storing prior usage data of the surgical instrument. By providing a simulation of the prior use of the surgical instrument, the cause of a particular occurrence (e.g., an alert, malfunction, activation or deactivation of particular function(s), etc.) can be readily determined, obviating the need to analyze and correlate textual data in order to determine the cause of a particular occurrence. Although detailed below with respect to particular systems, instruments, and/or components, the aspects and features of the present disclosure are equally applicable for use with any suitable surgical systems, instruments, and components.

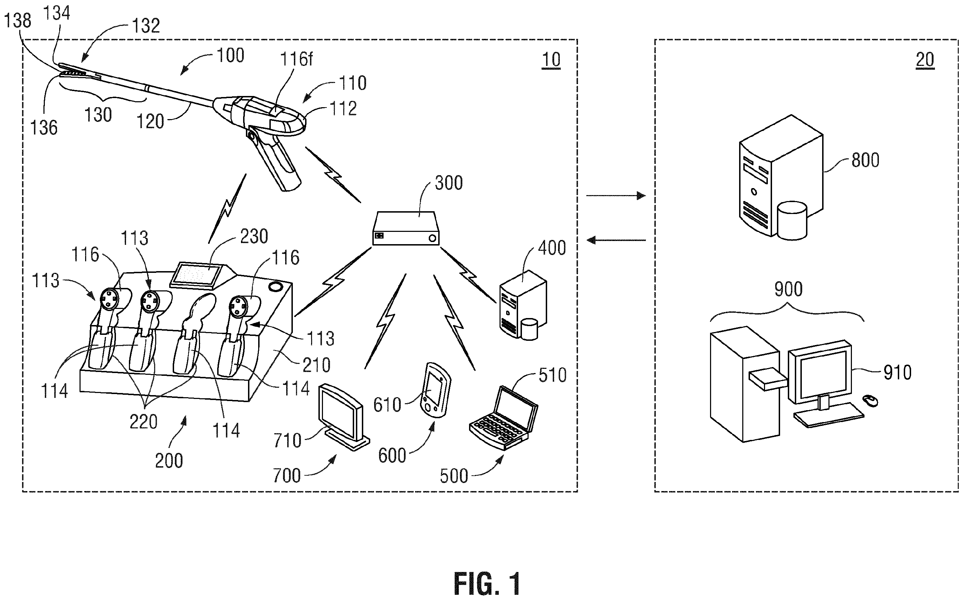

Referring to FIG. 1, a medical facility 10 is shown generally including one or more surgical instruments, e.g., an electromechanical surgical stapler 100, and a plurality of devices configured for use within medical facility 10, e.g., a charger 200, a router 300, a server 400, a computer 500, a smartphone or tablet 600, a display monitor 700, etc. Surgical stapler 100 and/or one or more of devices 200-700 may further be configured to communicate with a remote facility 20 that includes, for example, one or more servers 800 and/or one or more computers 900.

Surgical stapler 100 is an electromechanical, battery-powered device including a handle assembly 110 configured for selective connection with an adapter 120 which, in turn, is configured for selective connection with an end effector 130. Alternatively, an end effector may be directly connected to handle assembly 110. Handle assembly 110 includes an outer housing 112 that is configured to receive and enclose an inner assembly 113 (shown docked in charger 200 in FIG. 1). Inner assembly 113 includes a battery assembly 114 and a power-pack 116. Battery assembly 114 provides power to power-pack 116 for powering surgical stapler 100 while power-pack 116 controls the operation of surgical stapler 100. Adapter 120 interconnects power-pack 116 of handle assembly 110 and end effector 130 and enables mechanical and electrical communication therebetween.

End effector 130 includes a tool assembly 132 having an anvil assembly 134 and a cartridge assembly 136. Cartridge assembly 136 is pivotal in relation to anvil assembly 134, although this configuration may be revered, between an open position and a closed position and houses a staple cartridge 138 therein. Upon firing, staples (not shown) are sequentially ejected from staple cartridge 138 for formation against anvil assembly 134.

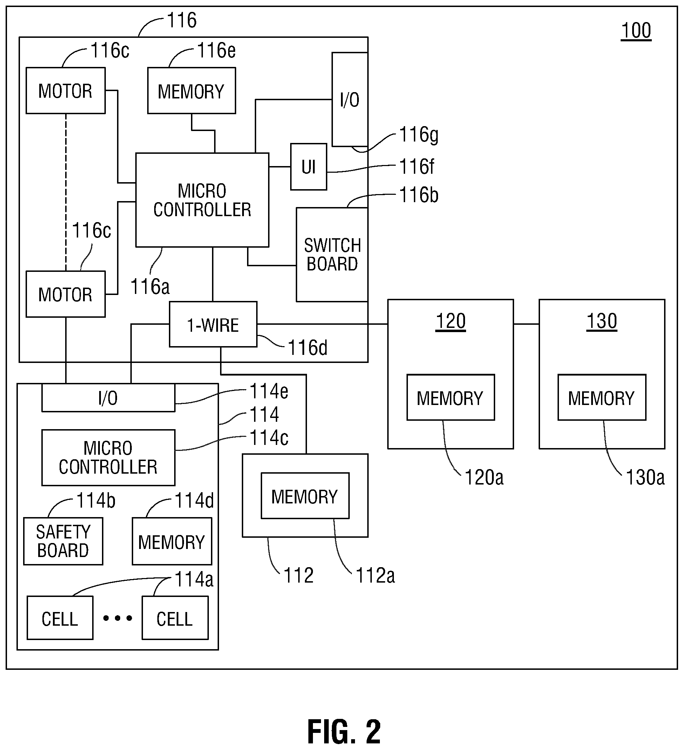

With additional reference to FIG. 2, as noted above, battery assembly 114 provides power to power-pack 116 to power surgical stapler 100. Battery assembly 114 generally includes a plurality of battery cells 114a, a safety board 114b, a microcontroller 114c, a memory 114d, and an input/output 114e. Safety board 114b monitors the charging and discharging of battery cells 114a to ensure safe and proper operation thereof. Microcontroller 114c controls the receipt of energy by battery cells 114a and the delivery of energy from battery cells 114a. Memory 114d stores information relating to battery assembly 114 such as identifying data, usage data, operating programs (for charging and/or discharging), etc. Identifying data may include the serial number, model number, manufacture date, etc. Usage data may include the charge status of battery assembly 114, the number of procedures in which battery assembly 114 has been used, the number of charge and/or discharge cycles battery assembly 114 has been through, voltage and/or current information during charging and/or discharging, the duration of a charge and/or discharge, event logging data, fault and error data, environmental data (e.g., temperature), etc. Input/output 114e enables data and power transmission to/from battery assembly 114.

Power-pack 116, as noted above, controls the operation of surgical stapler 100. Power-pack 116 generally includes a microcontroller 116a, a switch board 116b, a plurality of motors 116c, a 1-wire communication chip 116d, a memory 116e, a user interface 116f, and an input/output 116g. Microcontroller 116a controls the operation of surgical stapler 100 by communicating, via the 1-wire communication chip 116d, with battery assembly 114, outer housing 112, adapter 120, and end effector 130 of surgical stapler 100. Switch board 116b is coupled to user-operable buttons mounted on outer housing 112 and communicates with microcontroller 116a to effect operation of surgical stapler 100 in accordance with the particular button or buttons depressed by the user. Motors 116c, in response to signals received from microcontroller 116a via the 1-wire communication chip 116d and power received from battery assembly 114, drive the mechanical operations of surgical stapler 100 (e.g., to rotate, articulate, close, fire, and/or open end effector 130). Memory 116e stores information relating to inner assembly 113, e.g., battery assembly 114 and power-pack 116. Information regarding battery assembly 114 may be retrieved from memory 114d of battery assembly 114, or may be sensed therefrom. Information regarding power-pack 116 may include identifying data, usage data, operating programs, etc. Identifying data of power-pack 116 may include the serial number, model number, manufacture date, etc. Usage data of power-pack 116 may include, for example, keystroke data, event logging data, fault and error data, sensor feedback data (of surgical stapler 100 and/or tissue), operational data (e.g., of motors 116c), environmental data (e.g., temperature), etc. Memory 116e may further store information relating to outer housing 112, adapter 120, and/or end effector 130 such as identifying and usage data thereof, as detailed below.

User interface 116f is provided in the form of a display screen viewable from the exterior of outer housing 112 of handle assembly 110 and is configured to display information such as the operational status of surgical stapler 100, usage information associated with any or all of the components of surgical stapler 100, notifications, etc. Input/output 116g may be configured for wired connection and/or wireless communication with one or more external devices (e.g., charger 200, router 300, server 400, computer 500, smartphone or tablet 600, display monitor 700, etc.) for communicating status information, data, updates, notifications, etc. therebetween.

Each of outer housing 112, adapter 120, and end effector 130 of surgical stapler 100 includes a memory 112a, 120a, 130a, respectively, storing information such as identifying data (e.g., serial number, model number, manufacture date, etc.), usage data (e.g., data indicating connection to or disconnection from other component(s), operational data, fault data, sensor feedback data, tissue data, environmental data, etc.), and setting data. Utilizing the 1-wire communication chip 116d, microcontroller 116a of power-pack 116 is configured to access memories 112a, 120a, 130a when outer housing 112, adapter 120, and end effector 130, respectively, are attached to handle assembly 110, to enable retrieval of such information and/or storage of such information in memory 116e of power-pack 116.

With respect to identifying data microcontroller 116a is configured to read such identifying data from memories 112a, 120a, 130a of outer housing 112, adapter 120, and end effector 130, respectively, and store such information in memory 116e of power-pack 116 to enable tracking of that particular component, recognition upon subsequent attachment, and association of usage data with that particular component.

With respect to setting data microcontroller 116a is configured to read such setting data from memories 112a, 120a, 130a of outer housing 112, adapter 120, and end effector 130, respectively, store such information in memory 116e of power-pack 116, and match the setting data to a corresponding operating program stored in memory 116e of power-pack 116 to ensure compatible operation of power-pack 116 in accordance with the particular configuration(s) of the component(s) attached thereto.

With respect to usage data microcontroller 116a reads usage data from memories 112a, 120a, 130a of outer housing 112, adapter 120, and/or end effector 130, respectively, for storage in memory 116e of power-pack 116, together with the usage data of inner assembly 113. As an alternative or in addition to reading memories 112a, 120a, 130a, microcontroller 116a may directly receive usage data (e.g., in real-time) from the appropriate components of outer housing 112, adapter 120, and/or end effector 130. The usage data of inner assembly 113, outer housing 112, adapter 120, and end effector 130 may be time-stamped and/or correlated to the identification information of the corresponding component or components(s). This information is stored in memory 116e of power-pack 116 in one or more data files ("usage data files"), in any suitable format. As detailed below, these usage data files are configured to be transmitted to an external device that processes and outputs the data as a simulation of the prior use of surgical stapler 100. Microcontroller 116a may be configured to output the usage data files stored in memory 116e, via a wired or wireless connection, continuously, periodically, after each use or a number of uses, upon connection to an external device, upon connection or disconnection of component(s) of surgical stapler 100, upon request, etc.

Referring again to FIG. 1, surgical stapler 100 and one or more of the devices within medical facility 10 and/or one or more of the devices within remote facility 20 may be utilized to provide a simulation of prior use of the surgical stapler 100 based on the usage data files of surgical stapler 100. The configuration of surgical stapler 100 and the collection and storage of information to create the usage data files are detailed above. Each of the other devices of medical facility 10 as well as the devices of remote facility 20 will be generally described below, followed by a more detailed description of the use of such devices, in conjunction with the usage data files from surgical stapler 100, to provide a simulation of prior use of surgical stapler 100.

Charger 200 includes a base 210 defining a plurality of charging bays 220, each configured to receive a battery assembly 114 and a power-pack 116 (together as inner assembly 113 or separately from one another) of a surgical stapler 100, for enabling charging and/or data transfer therebetween. Charger 200 further includes a user interface 230 configured to display information relating to an attached battery assembly 114, power-pack 116, or other component of surgical stapler 100 that has been previously used with the power-pack 116. Charger 200 may further be configured to download information (e.g., the usage data files) from power-packs 116 connected thereto. Charger 200 may itself process the usage data files to provide a simulation of prior use of surgical stapler 100, or may store the data for subsequent transmission to a device configured to process the usage data files and provide a simulation based thereon. Charger 200 may include any suitable software, firmware, and hardware for the above-noted purposes.

Router 300 may be configured as a hub that relays local communications (e.g., between surgical stapler 100, charger 200, server 400, computer 500, smartphone or tablet 600, and/or display monitor 700), and/or remote communications (e.g., between any of the devices of medical facility 10 and any of the devices of remote facility 20). Server 400 may be configured to transmit/receive data to/from any of the devices of medical facility 10 and/or any of the devices of remote facility 20 and to store such data in a database, process the data, etc. Router 300 and server 400 may include any suitable software, firmware, and hardware for these purposes. Similarly as with charger 200, the usage data files of surgical stapler 100 may be relayed via router 300 and/or stored on server 400 such that the usage data files may ultimately be used by other device(s) to provide a simulation of the prior use of surgical stapler 100.

Computer 500, smartphone or tablet 600, and display monitor 700 may be configured to receive the usage data files from surgical stapler 100, charger 200, server 400, and/or one another (e.g., via router 300) and to process the usage data files to provide a simulation of the prior use of surgical stapler 100 based thereupon (e.g., on a respective display screen 510, 610, 710 thereof). Alternatively or additionally, computer 500, smartphone or tablet 600, and display monitor 700 may be configured to store the usage data files for subsequent transmission to one another and/or any of the devices of remote facility 20, for processing to provide a simulation of the prior use of surgical stapler 100. Devices 500, 600, 700 may include any suitable software, firmware, and hardware for these purposes.

Continuing with reference to FIG. 1, remote facility 20, as mentioned above, may include one or more servers 800 and one or more computers 900. Server 800 may be configured to receive the usage data files from any of the devices within medical facility 10 and/or to transmit data thereto. Server 800 may further be configured to store the usage data files, process the usage data files, and/or output the usage data files to computer 900 or another device. Computer 900 may be configured to receive, store, process, and/or transmit the usage data files locally and/or remotely. More specifically, computer 900 may be configured to process the usage data files of surgical stapler 100 to provide a simulation of the prior use of surgical stapler 100 (e.g., displayed on a display 910 of computer 900). Server 800 and computer 900 may include any suitable software, firmware, and hardware for these purposes.

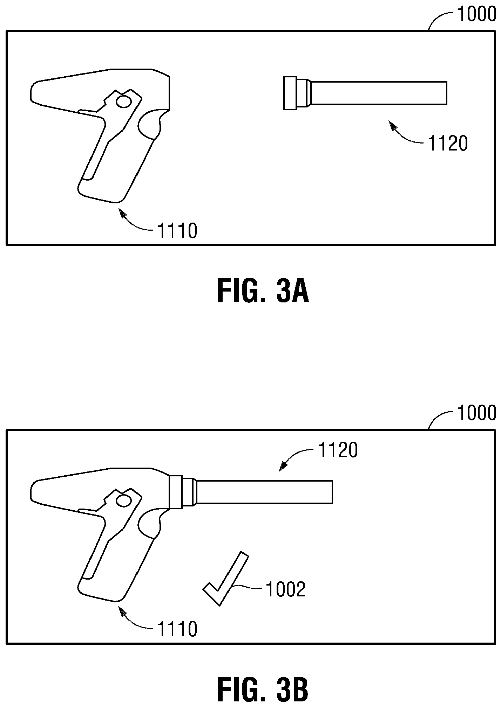

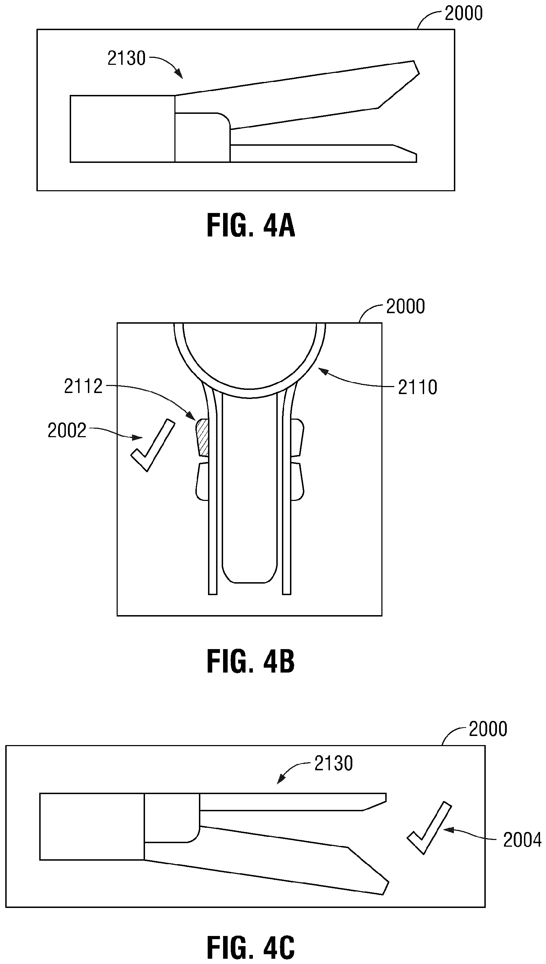

The devices (e.g., charger 200, server 400, computer 500, smartphone or tablet 600, server 800, and/or computer 900) configured to process the usage data files and provide a simulation of the prior use of surgical stapler 100 (e.g., to be displayed on display 510, 610, 710, 910, or other suitable display), include a processor and a storage unit storing instructions to be executed by the processor to order analyze (e.g., order, manipulate, correlate, etc.) the information stored in the usage data files; associate the same with an image or images; construct a simulation of the prior use of surgical stapler 100 based thereupon; and output the image simulation to a display for viewing by a user. Ordering, manipulating, correlating, etc. the information stored in the usage data files may include, for example: grouping related data (based upon type, cause/effect, and/or chronologically); ordering the grouped data; associating the grouped data to a particular function, cause, and/or result; etc. Associating the ordered, manipulated, and/or correlated data with an image or images may include, for example, determining an image or images that represent the function(s) or action(s) associated with that data. Outputting the image simulation may include ordering the images chronologically to form a chronological simulation of the prior use of surgical stapler 100. The output simulation may include a slideshow of still images, an animation based upon images, or other suitable visual representation. The above and other aspects and features of the present disclosure will become more apparent in view of the exemplary simulations detailed below with respect to FIGS. 3A-3D and 4A-4E. Although detailed with respect to generic displays 1000, 2000 (FIGS. 3A-3D and 4A-4E, respectively), the processing and display of these simulations may be provided on any one of the above-detailed devices and/or displays, or any other suitable device and/or display.

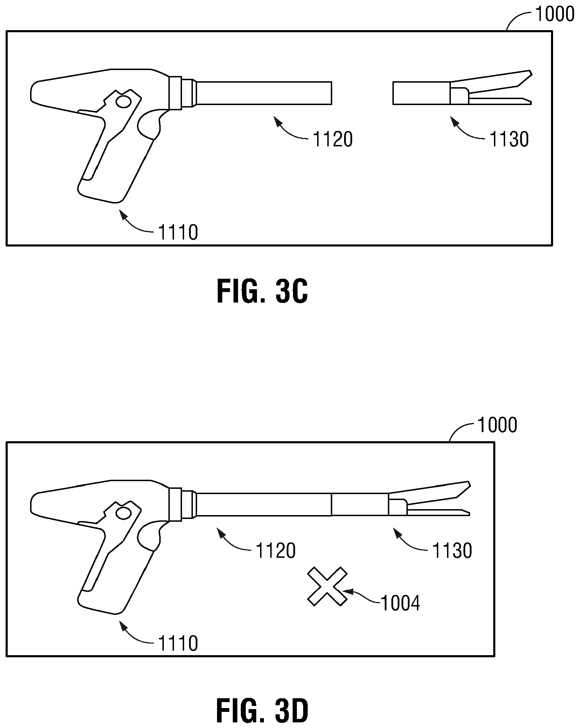

Referring to FIGS. 3A-3D, in conjunction with FIG. 1, a simulation, displayed on a display 1000, of the prior use of surgical stapler 100 and, more particularly, the assembly thereof, is detailed. FIG. 3A illustrates an image 1110 representing handle assembly 110 of surgical stapler 100 and, spaced-apart therefrom, an image 1120 representing adapter 120 of surgical stapler 100; FIG. 3B illustrates image 1110 and image 1120 in engagement with one another and an icon 1002 indicating a successful and proper connection therebetween; FIG. 3C illustrates the engaged images 1110, 1120 and an image 1130, spaced-apart therefrom, representing end effector assembly 130 of surgical stapler 100; and FIG. 3D illustrates images 1110, 1120, and 1130 in engagement with one another and an icon 1004 indicating an unsuccessful or improper connection between adapter 120 and end effector 130.

When viewed together, FIGS. 3A-3D illustrate, initially, the successful and proper connection of adapter 120 with handle assembly 110 (represented by the approximation and engagement of images 1110, 1120 and icon 1002) and, subsequently, the unsuccessful or improper connection of end effector 130 with adapter 12 (represented by the approximation and engagement of images 1130 with images 1110, 1120 and icon 1004). FIGS. 3A-3D may be consecutive images in a slideshow or may be snapshots during the course of an animation showing the approximation and connection of adapter 120 with handle assembly 110 and, thereafter, the approximation and connection of end effector 130 with the connected adapter 120.

The underlying data from the usage data files of surgical stapler 100 used to construct the simulation with respect to the connection of adapter 120 with handle assembly 110 (FIGS. 3A and 3B) may include: data indicating a successful mechanical connection of adapter 120 with handle assembly 110, data indicating that adapter 120 has been verified as being compatible for use with handle assembly 110, data indicating that adapter 120 has not yet reached any use limits associated therewith; and data indicating that adapter 120 has been successfully calibrated.

The underlying data from the usage data files of surgical stapler 100 used to construct the simulation with respect to the connection of end effector 130 with adapter 120 (FIGS. 3C and 3D) may include: data indicating at least one error with respect to the mechanical connection of end effector 130 with adapter 120, verification of end effector 130 as being compatible for use with handle assembly 110 and/or adapter 120, a use limit(s) of end effector 130, or calibration of end effector 130.

It is understood that the simulation represented in FIGS. 3A-3D is simplified for illustrating purposes and that more detailed images and/or icons identifying the particular error(s) that caused an unsuccessful or improper connection may be provided. As can be appreciated in view of the simulation illustrated in FIGS. 3A-3D, a user reviewing the simulation, for example, would be readily able to determine that it was the unsuccessful or improper connection of end effector 130 with adapter 120 that inhibited further use of surgical stapler 100. Further, a split-screen display may be provided such that, in conjunction with illustrating the error(s) that caused the unsuccessful or improper connection, a simulation representing a successful and proper connection may be provided, thus further enhancing the user's ability to understand the error(s).

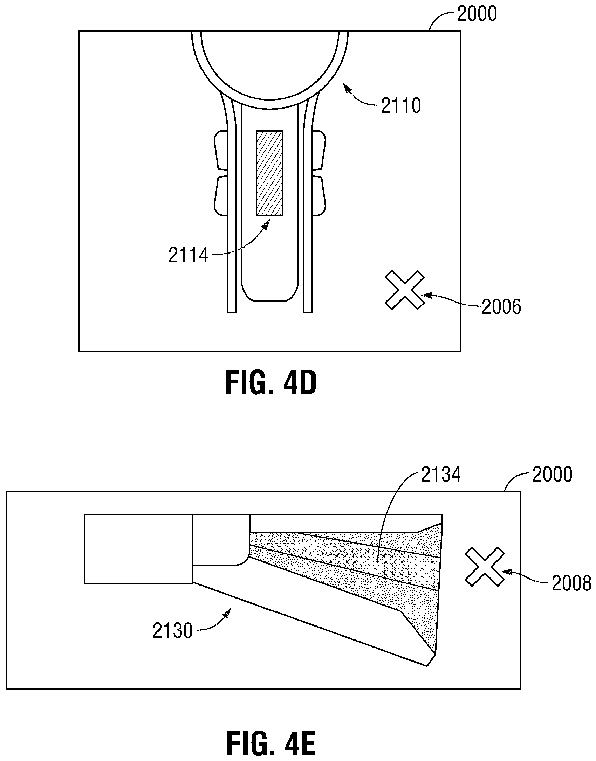

Referring to FIGS. 4A-4E, in conjunction with FIG. 1, a simulation, displayed on a display 2000, of the prior use of surgical stapler 100 and, more particularly, the use thereof during a surgical procedure, is detailed. FIG. 4A illustrates an image 2130 representing end effector 130 of surgical stapler 100, disposed in a first orientation; FIG. 4B illustrates an image 2110 of a portion of handle assembly 110 of surgical stapler 100 including a highlighted portion 2112 representing that a rotation button of the handle assembly 110 has been actuated, and an icon 2002 indicating that rotation, in response to actuation of the rotation button, was successful; FIG. 4C illustrates the image 2130 representing end effector 130 of surgical stapler 100, disposed in a second orientation resulting from the rotation thereof in response to actuation of the rotation button, and an icon 2004 indicating that rotation of the end effector 130 was successful; FIG. 4D illustrates the image 2110 of the portion of handle assembly 110 of surgical stapler 100 including a highlighted portion 2114 representing that a clamping button of the handle assembly 110 has been actuated, and an icon 2006 indicating that clamping, in response to actuation of the clamping button, was not successful; and FIG. 4E illustrates the image 2130 representing end effector 130 of surgical stapler 100, an image 2132 representing a relatively larger volume of tissue that end effector 130 is attempting to clamp, and an icon 1108 indicating that clamping the larger volume of tissue was not successful.

When viewed together, FIGS. 4A-4E illustrate, initially, the rotation of end effector 130 to a desired orientation in response to actuation of a rotation button associated with handle assembly 110, followed by the attempted, but unsuccessful clamping of a relatively larger volume of tissue with end effector 130. Similarly as detailed above, FIGS. 4A-4E may be consecutive images in a slideshow or may be snapshots during the course of an animation showing the rotation and subsequent attempted clamping of end effector 130.

The underlying data from the usage data files of surgical stapler 100 used to construct the simulation with respect to the rotation of end effector 130 in response to actuation of the rotation button of handle assembly 110 (FIGS. 4A-4C) may include: data indicating an initial orientation of end effector 130, data indicating an actuation of the rotation button, data indicating driving of one or more of motors 116c to effect the rotation of end effector 130, and data indicating the resultant position of end effector 130.

The underlying data from the usage data files of surgical stapler 100 used to construct the simulation with respect to the attempted clamping of the large volume of tissue with end effector 130 (FIGS. 4D and 4E) may include: data indicating that end effector 130 is disposed in an open condition; data indicating an actuation of the clamping button, data indicating driving of one or more of motors 116c to attempt to clamp tissue with end effector 130, data indicating a resistance to further clamping (based upon, for example, too large a load for the one or more of motors 116c, feedback from a strain gauge, feedback from a force sensor, etc.), data indicating that the one or more motors 116c have been stopped, and data indicating the spacing between anvil assembly 134 and a cartridge assembly 136 of end effector 130 is outside the acceptable range for the clamping position.

It is understood that the simulation represented in FIGS. 4A-4E is simplified for illustrating purposes and that more detailed images and/or icons identifying the particular error(s) that caused an unsuccessful or improper connection may be provided. As can be appreciated in view of the simulation illustrated in FIGS. 4A-4E, a user reviewing the simulation, for example, would be readily able determine that clamping was unsuccessful due to the presence of too much tissue between anvil assembly 134 and a cartridge assembly 136 of end effector 130.

Surgical systems such as the surgical stapler described herein may also be configured to work with robotic surgical systems and what is commonly referred to as "Telesurgery." Such robotic surgical systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

## Claims

1. A surgical system (10,20), comprising:
a surgical instrument (100) including a memory (116e) configured to store usage data from a prior use of the surgical instrument in at least one usage data file;
a processing component (900, 800, 700, 600, 500, 400) configured to receive the at least one usage data file from the surgical instrument, the processing component configured to generate a simulation (1000, 2000) of the prior use of the surgical instrument based upon the at least one usage data file; and
a display (610, 510, 710, 910) component operably coupled to the processing component and configured to visually display the simulation of the prior use of the surgical instrument.
wherein the simulation associates the usage data with an image or images of the surgical instrument.

2. The surgical system according to claim 1, wherein the usage data stored in the at least one usage data file is time-stamped; and/or wherein the usage data includes at least one of keystroke data, sensor feedback data, or operational data.

3. The surgical system according to claim 1 or claim 2, wherein the surgical instrument includes a plurality of components configured to releasably engage one another, each of the plurality of components configured to generate component usage data for storage in the at least one usage data file; preferably wherein the component usage data is associated, in the at least one usage data file, with identifying data of the component thereof.

4. The surgical system according to any preceding claim, wherein the processing component and the display component are associated with a computer.

5. The surgical system according to any preceding claim, wherein the simulation is visually displayed, via the display component, as a slideshow and/or wherein the simulation is visually displayed, via the display component, as an animation.

6. The surgical system according to any preceding claim, wherein the simulation further includes at least one icon indicating a proper or improper event in the prior use of the surgical instrument.

7. The surgical system according to any preceding claim, wherein the processing component is configured to at least one of group, manipulate, or correlate the usage data stored in the at least one usage data file; preferably wherein the processing component is configured to associate the at least one of grouped, manipulated, or correlated usage data with the image or images used to generate the simulation.

8. A method, comprising:
obtaining usage data from a prior use of a surgical instrument;
storing the usage data in at least one usage data file;
transmitting the at least one usage data file to a processor;
generating at the processor a simulation of the prior use of the surgical instrument based upon the at least one usage data file; and
displaying the simulation of the prior use of the surgical instrument,
wherein the simulation associates the usage data with an image or images of the surgical instrument.

9. The method according to claim 8, wherein storing the usage data in the at least one usage data file includes time-stamping the usage data.

10. The method according to claim 8 or claim 9, wherein obtaining the usage data includes obtaining at least one of keystroke data, sensor feedback data, or operational data.

11. The method according to any of claims 8 to 10, wherein obtaining the usage data includes obtaining component usage data from a plurality of components of the surgical instrument.

12. The method according to claim 11, wherein storing the usage data includes associating the component usage data with identifying data of the component thereof.

13. The method according to any of claims 8 to 12, wherein displaying the simulation includes displaying a slideshow; and/or wherein displaying the simulation includes displaying an animation.

14. The method according to any of claims 8 to 13, wherein displaying the simulation further includes displaying an icon indicating a proper or improper event in the prior use of the surgical instrument.

15. The method according to any of claims 8 to 14, wherein generating the simulation includes:
at least one of grouping, manipulating, or correlating the usage data stored in the at least one usage data file; and
associating the at least one of grouped, manipulated, or correlate usage data with the image or images of the surgical instrument.

## Patentansprüche

1. Chirurgisches System (10, 20), das Folgendes umfasst:
ein chirurgisches Instrument (100), das einen Speicher (116e) einschließt, der konfiguriert ist, um Verwendungsdaten von einer früheren Verwendung des chirurgischen Instruments in mindestens einer Verwendungsdatendatei zu speichern;
eine Verarbeitungskomponente (900, 800, 700, 600, 500, 400), die konfiguriert ist, um die mindestens eine Verwendungsdatendatei von dem chirurgischen Instrument zu empfangen, wobei die Verarbeitungskomponente konfiguriert ist, um eine Simulation (1000, 2000) der früheren Verwendung des chirurgischen Instruments basierend auf der mindestens einen Verwendungsdatendatei zu erzeugen; und
eine Darstellungskomponente (610, 510, 710, 910), die betriebsfähig mit der Verarbeitungskomponente gekoppelt und konfiguriert ist, um die Simulation der früheren Verwendung des chirurgischen Instruments visuell darzustellen, wobei die Simulation die Verwendungsdaten einem Bild oder Bildern des chirurgischen Instruments zuordnet.

2. Chirurgisches System nach Anspruch 1, wobei die Verwendungsdaten, die in der mindestens einen Verwendungsdatendatei gespeichert sind, zeitgestempelt sind; und/oder
wobei die Verwendungsdaten mindestens eines von Tastendruckdaten, Sensorrückmeldedaten oder Betriebsdaten einschließen.

3. Chirurgisches System nach Anspruch 1 oder Anspruch 2, wobei das chirurgische Instrument mehrere Komponenten einschließt, die konfiguriert sind, um lösbar miteinander einzugreifen, wobei jede der mehreren Komponenten konfiguriert ist, um Komponentenverwendungsdaten für die Speicherung in der mindestens einen Verwendungsdatendatei zu erzeugen;
wobei vorzugsweise die Komponentenverwendungsdaten in der mindestens einen Verwendungsdatendatei den Identifikationsdaten der Komponente davon zugeordnet sind.

4. Chirurgisches System nach einem vorangehenden Anspruch, wobei die Verarbeitungskomponente und die Darstellungskomponente einem Computer zugeordnet sind.

5. Chirurgisches System nach einem vorangehenden Anspruch, wobei die Simulation visuell über die Darstellungskomponente als eine Diaschau dargestellt wird und/oder wobei die Simulation visuell über die Darstellungskomponente als eine Animation dargestellt wird.

6. Chirurgisches System nach einem vorangehenden Anspruch, wobei die Simulation ferner mindestens ein Symbol einschließt, das ein ordnungsgemäßes oder nicht ordnungsgemäßes Ereignis bei der früheren Verwendung des chirurgischen Instruments anzeigt.

7. Chirurgisches System nach einem vorangehenden Anspruch, wobei die Verarbeitungskomponente konfiguriert ist, um die in der mindestens einen Verwendungsdatendatei gespeicherten Verwendungsdaten mindestens zu gruppieren, zu manipulieren oder zu korrelieren;
wobei die Verarbeitungskomponente vorzugsweise konfiguriert ist, um mindestens eine aus den gruppierten, manipulierten oder korrelierten Verwendungsdaten dem Bild oder den Bildern zuzuordnen, die zum Erzeugen der Simulation verwendet werden.

8. Verfahren, das Folgendes umfasst:
Erhalten von Verwendungsdaten aus einer früheren Verwendung eines chirurgischen Instruments;
Speichern der Verwendungsdaten in mindestens einer Verwendungsdatendatei;
Übertragen der mindestens einen Verwendungsdatendatei an einen Prozessor;
Erzeugen einer Simulation der früheren Verwendung des chirurgischen Instruments am Prozessor basierend auf der mindestens einen Verwendungsdatendatei; und
Darstellen der Simulation der früheren Verwendung des chirurgischen Instruments, wobei die Simulation die Verwendungsdaten einem Bild oder Bildern des chirurgischen Instruments zuordnet.

9. Verfahren nach Anspruch 8, wobei das Speichern der Verwendungsdaten in der mindestens einen Verwendungsdatendatei das Zeitstempeln der Verwendungsdaten einschließt.

10. Verfahren nach Anspruch 8 oder Anspruch 9, wobei das Erhalten der Verwendungsdaten das Erhalten von mindestens einem von Tastendruckdaten, Sensorrückmeldedaten oder Betriebsdaten einschließt.

11. Verfahren nach einem der Ansprüche 8 bis 10, wobei das Erhalten der Verwendungsdaten das Erhalten von Komponentenverwendungsdaten von mehreren Komponenten des chirurgischen Instruments einschließt.

12. Verfahren nach Anspruch 11, wobei das Speichern der Verwendungsdaten das Zuordnen der Komponentenverwendungsdaten zu Identifizierungsdaten der Komponente davon einschließt.

13. Verfahren nach einem der Ansprüche 8 bis 12, wobei das Darstellen der Simulation das Darstellen einer Diaschau einschließt; und/oder
wobei das Darstellen der Simulation das Darstellen einer Animation einschließt.

14. Verfahren nach einem der Ansprüche 8 bis 13, wobei das Darstellen der Simulation ferner das Darstellen eines Symbols einschließt, das ein ordnungsgemäßes oder nicht ordnungsgemäßes Ereignis bei der früheren Verwendung des chirurgischen Instruments darstellt.

15. Verfahren nach einem der Ansprüche 8 bis 14, wobei das Erzeugen der Simulation Folgendes einschließt:
mindestens eines von Gruppieren, Manipulieren oder Korrelieren der Verwendungsdaten, die in der mindestens einen Verwendungsdatendatei gespeichert sind; und
Zuordnen von mindestens einem von den gruppierten, manipulierten oder korrelierten Verwendungsdaten zu dem Bild oder den Bildern des chirurgischen Instruments.

## Revendications

1. Système chirurgical (10, 20), comprenant :
un instrument chirurgical (100) comportant une mémoire (116e) configurée pour stocker des données d'utilisation provenant d'un usage antérieur de l'instrument chirurgical dans au moins un fichier de données d'utilisation ;
un composant de traitement (900, 800, 700, 600, 500, 400) configuré pour recevoir l'au moins un fichier de données d'utilisation à partir de l'instrument chirurgical, le composant de traitement étant configuré pour générer une simulation (1000, 2000) de l'usage antérieur de l'instrument chirurgical sur la base de l'au moins un fichier de données d'utilisation ; et
un composant d'affichage (610, 510, 710, 910) couplé fonctionnellement au composant de traitement et configuré pour afficher visuellement la simulation de l'usage antérieur de l'instrument chirurgical, la simulation associant les données d'utilisation à une image ou à des images de l'instrument chirurgical.

2. Système chirurgical selon la revendication 1, dans lequel les données d'utilisation stockées dans l'au moins un fichier de données d'utilisation sont horodatées ; et/ou
les données d'utilisation comportant au moins les unes parmi des données de frappe, des données de retour d'informations du capteur ou des données opérationnelles.

3. Système chirurgical selon la revendication 1 ou la revendication 2, dans lequel l'instrument chirurgical comporte une pluralité de composants configurés pour venir en prise de manière amovible les uns avec les autres, chacun de la pluralité de composants étant configuré pour générer des données d'utilisation de composant destinées au stockage dans l'au moins un fichier de données d'utilisation ;
les données d'utilisation de composant étant de préférence associées, dans l'au moins un fichier de données d'utilisation, à des données d'identification du composant de celles-ci.

4. Système chirurgical selon une quelconque revendication précédente, dans lequel le composant de traitement et le composant d'affichage sont associés à un ordinateur.

5. Système chirurgical selon une quelconque revendication précédente, dans lequel la simulation est affichée visuellement, par l'intermédiaire du composant d'affichage, sous forme de diaporama et/ou la simulation étant affichée visuellement, par l'intermédiaire du composant d'affichage, sous forme d'animation.

6. Système chirurgical selon une quelconque revendication précédente, dans lequel la simulation comporte en outre au moins une icône indiquant un événement correct ou incorrect lors de l'usage antérieur de l'instrument chirurgical.

7. Système chirurgical selon une quelconque revendication précédente, dans lequel le composant de traitement est configuré pour exécuter, au moins une action parmi, grouper, manipuler ou corréler des données d'utilisation stockées dans l'au moins un fichier de données d'utilisation ;
le composant de traitement étant de préférence configuré pour associer au moins les unes parmi des données d'utilisation groupées, manipulées ou corrélées à l'image ou aux images utilisées pour générer la simulation.

8. Procédé, comprenant :
l'obtention de données d'utilisation à partir d'un usage antérieur d'un instrument chirurgical ;
le stockage des données d'utilisation dans au moins un fichier de données d'utilisation ;
la transmission de l'au moins un fichier de données d'utilisation à un processeur ;
la génération au niveau du processeur d'une simulation de l'usage antérieur de l'instrument chirurgical sur la base de l'au moins un fichier de données d'utilisation ; et
l'affichage de la simulation de l'usage antérieur de l'instrument chirurgical, la simulation associant les données d'utilisation à une image ou des images de l'instrument chirurgical.

9. Procédé selon la revendication 8, dans lequel le stockage des données d'utilisation dans l'au moins un fichier de données d'utilisation comporte l'horodatage des données d'utilisation.

10. Procédé selon la revendication 8 ou la revendication 9, dans lequel l'obtention des données d'utilisation comporte l'obtention d'au moins les unes parmi des données de frappe, des données de retour d'informations du capteur ou des données opérationnelles.

11. Procédé selon l'une quelconque des revendications 8 à 10, dans lequel l'obtention des données d'utilisation comporte l'obtention de données d'utilisation de composant à partir d'une pluralité de composants de l'instrument chirurgical.

12. Procédé selon la revendication 11, dans lequel le stockage des données d'utilisation comporte l'association des données d'utilisation de composant aux données d'identification du composant de celles-ci.

13. Procédé selon l'une quelconque des revendications 8 à 12, dans lequel l'affichage de la simulation comporte l'affichage d'un diaporama ; et/ou
l'affichage de la simulation comportant l'affichage d'une animation.

14. Procédé selon l'une quelconque des revendications 8 à 13, dans lequel l'affichage de la simulation comporte en outre l'affichage d'une icône indiquant un événement correct ou incorrect lors de l'usage antérieur de l'instrument chirurgical.

15. Procédé selon l'une quelconque des revendications 8 à 14, dans lequel la génération de la simulation comporte :
au moins un élément parmi le groupement, la manipulation ou la corrélation des données d'utilisation stockées dans l'au moins un fichier de données d'utilisation ; et
l'association des au moins unes parmi les données d'utilisation groupées, manipulées ou corrélées à l'image ou aux images de l'instrument chirurgical.
